(19) Europäisches Patentamt
European Patent Office
Office européen des brevets

(11) EP 4 455 194 A1

(12) EUROPEAN PATENT APPLICATION
published in accordance with Art. 153(4) EPC

(43) Date of publication:
**30.10.2024 Bulletin 2024/44**

(21) Application number: **23892047.4**

(22) Date of filing: **16.11.2023**

(51) International Patent Classification (IPC):
***C08J 3/24*** (2006.01) ***C08J 3/12*** (2006.01)
***C08J 3/075*** (2006.01) ***C08F 220/04*** (2006.01)
***A61L 15/60*** (2006.01)

(52) Cooperative Patent Classification (CPC):
**A61L 15/60; C08F 220/04; C08J 3/075; C08J 3/12; C08J 3/24**

(86) International application number:
**PCT/KR2023/018487**

(87) International publication number:
**WO 2024/106984 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.11.2022 KR 20220153918**

(71) Applicant: **LG Chem, Ltd.**
**Yeongdeungpo-gu**
**Seoul 07336 (KR)**

(72) Inventors:
- **LEE, Hyemin**
  **Daejeon 34122 (KR)**
- **DO, Yun Kyung**
  **Daejeon 34122 (KR)**
- **HAN, Chang Hun**
  **Daejeon 34122 (KR)**
- **NAM, Hyemi**
  **Daejeon 34122 (KR)**

(74) Representative: **Plasseraud IP**
**104 Rue de Richelieu**
**CS92104**
**75080 Paris Cedex 02 (FR)**

# (54) SUPERABSORBENT POLYMER AND PREPARATION METHOD THEREOF

(57) The present disclosure relates to a super absorbent polymer and a preparation method of the same. More specifically, it relates to a super absorbent polymer having excellent absorption rate and absorption performance while exhibiting excellent rewet properties even without containing pulp, and a preparation method of the same.



Processed by Luminess, 75001 PARIS (FR)

## Description

[TECHNICAL FIELD]

Cross-reference to Related Application(s)

This application claims the benefit of Korean Patent Applications No. 10-2022-0153918 filed on November 16, 2022 and No. 10-2023-0159227 filed on November 16, 2023 with the Korean Intellectual Property Office, the disclosures of which are incorporated herein by reference in their entirety.

The present disclosure relates to a super absorbent polymer and a preparation method of the same. More specifically, it relates to a super absorbent polymer having excellent absorption rate and absorption performance while exhibiting excellent rewet properties even without containing pulp, and a preparation method of the same.

[BACKGROUND OF ART]

A super absorbent polymer (SAP) is a type of synthetic polymeric material capable of absorbing 500 to 1000 times its own weight of moisture. Various manufacturers have denominated it with different names, such as SAM (Super Absorbency Material), AGM (Absorbent Gel Material), and the like. Such super absorbent polymers started to be practically applied in sanitary articles, and they are now being widely used for water retaining soil products for gardening, water stop materials for the civil engineering and construction, sheets for raising seedling, fresh-keeping agents for food distribution fields, materials for poultices, or the like.

These super absorbent polymers have been widely used in the field of hygiene articles such as diapers or sanitary pads. In such hygiene articles, the super absorbent polymer is generally contained in a state of being spread in the pulp. In recent years, however, continuous efforts have been made to provide hygiene articles such as diapers having a thinner thickness. As a part of such efforts, the development of so-called pulpless diapers and the like in which the pulp content is reduced or pulp is not used at all is being actively advanced.

In the case of the above-described hygiene articles in which the pulp content is reduced or pulp is not used such as pulpless diapers, the rewet performance deteriorates as the absorption of liquids such as urine slows down. Therefore, the super absorbent polymer is required to exhibit high absorption performance and fast absorption rate because it must not only act as an absorbent body that absorbs liquid but also act as a pulp.

Accordingly, there is a continuous demand for the development of super absorbent polymers with excellent rewet properties while maintaining the basic absorption performance and fast absorption rate of super absorbent polymers.

[DETAILED DESCRIPTION OF THE INVENTION]

[Technical Problem]

The present disclosure relates to a super absorbent polymer having excellent absorption rate and absorption performance while exhibiting excellent rewet properties even without containing pulp, and a preparation method of the same.

[Technical Solution]

In order to solve the above problems, there is provided a super absorbent polymer including a base resin including a cross-linked polymer of an acrylic acid-based monomer having at least partially neutralized acidic groups and an internal cross-linking agent; and

a surface cross-linked layer formed by further cross-linking the cross-linked polymer using a surface cross-linking agent on the base resin,
wherein the super absorbent polymer satisfies the following conditions 1) to 4):

1) a centrifugal retention capacity (CRC) measured according to EDANA NWSP 241.0.R2 (15) is 32 g/g to 40 g/g,
2) an extractable content measured after swelling for 16 hours according to EDANA NWSP 270.0.R2 (15) is 10 wt% or less,
3) a vortex time by the vortex method is 30 seconds or less, and
4) a bulk density measured according to EDANA NWSP 251.0.R2 (15) is 0.55 g/ml or more and 0.65 g/ml or less.

There is also provided a preparation method of a super absorbent polymer in cluding

a step of forming a hydrogel polymer by polymerizing a monomer composition containing an acrylic acid-based monomer having at least partially neutralized acidic groups, an internal cross-linking agent, an initiator, and hydrophobic particles (step 1);
a step of forming a base resin powder by drying the hydrogel polymer (step 2);
a first pulverization step of pulverizing the base resin powder to include 50 wt% or more of particles with a particle diameter of 710 $\mu$m or more (step 3);
a second pulverization step of pulverizing the base resin powder obtained after the first pulverization step to include 10 wt% or less of particles with a particle diameter of 710 $\mu$m or more (step 4); and
a step of cross-linking the surface of the base resin in the presence of a surface cross-linking agent (step 5).

[ADVANTAGEOUS EFFECTS]

According to the present disclosure, when limiting the bulk density to a certain range by controlling the content of each particle size through pulverization, it is possible to provide a super absorbent polymer having excellent rewet properties while exhibiting an equal or better absorption rate and absorption capacity compared to conventional super absorbent polymers even without containing pulp.

[DETAILED DESCRIPTION OF THE EMBODIMENTS]

The terminology used herein is for the purpose of describing particular embodiments only and is not intended to limit the invention. The singular forms are intended to include the plural forms as well, unless the context clearly indicates otherwise. It will be further understood that the terms "include", "have", or "possess" when used in this specification, specify the presence of stated features, steps, components, or combinations thereof, but do not preclude the presence or addition of one or more other features, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

In addition, the terminologies are used merely to refer to specific embodiments, and are not intended to restrict the present disclosure. Singular expressions of the present disclosure may include plural expressions unless they are differently expressed contextually.

The terminology "polymer" in the present disclosure is in a state in which a water-soluble ethylene-based unsaturated monomer is polymerized, and may include all moisture content ranges, or all particle diameter ranges. Among the polymers, a polymer having a moisture content of about 40 wt% or more after polymerization and before drying may be referred to as a hydrogel polymer, and particles in which the hydrogel polymer is pulverized and dried may be referred to as a cross-linked polymer.

In addition, the terminology "super absorbent polymer particle" refers to a particulate material containing a cross-linked polymer in which an acrylic acid-based monomer having at least partially neutralized acidic groups is polymerized and cross-linked by an internal cross-linking agent.

In addition, the terminology "super absorbent polymer" is used to encompass all of a cross-linked polymer in which an acrylic acid-based monomer having at least partially neutralized acidic groups is polymerized or a base resin in the form of powder consisting of super absorbent polymer particles in which the cross-linked polymer is pulverized, and the cross-linked polymer or the base resin further processed, for example, surface cross-linking, fine reassembling, drying, pulverization, classification, etc., to be in a state suitable for commercialization, depending on the context. Accordingly, the terminology "super absorbent polymer " may be interpreted as including a plurality of super absorbent polymer particles.

In addition, the terminology "average diameter" of pores refers to a median of the longest diameter values of each of the plurality of pores included in the super absorbent polymer. This is to make it less affected by outliers compared to the simple average.

As the demand for thin and light diapers increases, research on pulpless diapers that do not contain pulp is underway. In pulpless diapers, the super absorbent polymer (SAP) acts as pulp, but an absorbent core made of 100% super absorbent polymer has the problem of slowing urine absorption and deteriorating rewet performance. Therefore, a super absorbent polymer having good absorption performance and absorption rate while exhibiting excellent rewet performance should be provided.

Accordingly, the present inventors have confirmed that the super absorbent polymer having a bulk density within a certain range formed by foaming using hydrophobic particles in a neutralizing solution and two pulverization

processes has improved rewet properties while exhibiting excellent absorption performance and absorption rate, thereby completing the present invention.

Hereinafter, the super absorbent polymer and the preparation method of the super absorbent polymer will be described in more detail step by step according to specific embodiments of the present invention.

**The super absorbent polymer**

The super absorbent polymer according to one embodiment includes a base resin including a cross-linked polymer of an acrylic acid-based monomer having at least partially neutralized acidic groups and an internal cross-linking agent; and a surface cross-linked layer formed by further cross-linking the cross-linked polymer using a surface cross-linking agent on the base resin.

The super absorbent polymer may have a centrifugal retention capacity (CRC) of 32 g/g to 40 g/g, as measured according to EDANA NWSP 241.0.R2 (15). Preferably, the centrifugal retention capacity of the super absorbent polymer may be 32 g/g or more, 33 g/g or more, or 33.4 g/g or more, and 40 g/g or less, 39 g/g or less, or 38.7 g/g or less. The higher the centrifugal retention capacity of the super absorbent polymer, the higher the absorbency of the diaper when used in diapers, which has the advantage of allowing less super absorbent polymer to be used. However, it is difficult to satisfy the centrifugal retention capacity of 40 g/g or more while maintaining a fast absorption rate. When the centrifugal retention capacity of the super absorbent polymer is 32 g/g to 40 g/g, both the centrifugal retention capacity and the absorption rate may be satisfactory.

The super absorbent polymer may have an extractable content of 10 wt% or less, as measured after swelling for 16 hours according to EDANA NWSP 270.0.R2 (15). Preferably, the super absorbent polymer may have an extractable content of 9 wt% or less, 8 wt% or less, or 7.7 wt% or less, and 1 wt% or more, 3 wt% or more, or 5 wt% or more, as measured after swelling for 16 hours.

The super absorbent polymer may have an absorption rate (vortex time) by the vortex method of 30 seconds or less. Preferably, it may be 29 seconds or less, or 28 seconds or less. As the lower vortex time can be evaluated as the better, the lower limit is theoretically 0 seconds, but may be about 10 seconds or more, 15 seconds or more, 18 seconds or more, 20 seconds or more, 21 seconds or more, or 22 seconds or more. When the absorption rate of the super absorbent polymer exceeds 30 seconds, there is a problem in that rewet performance is also reduced, and the super absorbent polymer with an absorption rate of less than 10 seconds is difficult to manufacture. The method of measuring the absorption rate of the super absorbent polymer will be described in more detail in Experimental Examples to be described later.

The super absorbent polymer may have a bulk density of 0.55 g/ml or more and 0.65 g/ml or less, as measured according to EDANA NWSP 251.0.R2 (15). Preferably, the bulk density of the super absorbent polymer may be 0.56 g/ml or more, 0.57 g/ml or more, or 0.58 g/ml or more, and 0.63 g/ml or less, 0.62 g/ml or less, or 0.60 g/ml or less.

Since a fast absorption rate means that the specific surface area of the particles is large, super absorbent polymer particles with a fast absorption rate have a low bulk density. If the bulk density is low, a large amount of super absorbent polymer is required, increasing manufacturing costs, so it is desirable for the bulk density to be moderately high. Considering the balance with the absorption rate, the above range of 0.55 g/ml or more and 0.65 g/ml or less is appropriate. When the bulk density exceeds 0.65 g/ml, flowability may be reduced.

The acrylic acid-based monomer is a compound represented by the following Chemical Formula 1:

[Chemical Formula1] $R^1$-$COOM^1$

in Chemical Formula 1,

$R^1$ is a C2 to C5 alkyl group having an unsaturated bond, and
$M^1$ is a hydrogen atom, a monovalent or divalent metal, an ammonium group, or an organic amine salt.

Preferably, the acrylic acid-based monomer may include at least one selected from the group consisting of acrylic acid, methacrylic acid, and a monovalent metal salt, a divalent metal salt, an ammonium salt and an organic amine salt thereof.

Herein, the acrylic acid-based monomers may be those having acidic groups which are at least partially neutralized. Preferably, the acrylic acid-based monomer partially neutralized with an alkali substance such as sodium hydroxide, potassium hydroxide, ammonium hydroxide, or the like may be used. A degree of neutralization of the acrylic acid-based monomer may be 40 to 95 mol%, 40 to 80 mol %, or 45 to 75 mol%. The range of the degree of neutralization can be adjusted according to final properties. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties,

such as those of an elastic rubber.

In addition, a concentration of the acrylic acid-based monomer may be about 20 to 60 wt%, or about 40 to 50 wt% based on the monomer composition containing the raw materials of the super absorbent polymer and the solvent, and properly controlled in consideration of polymerization time and reaction conditions. When the concentration of the monomer is excessively low, the yield of the super absorbent polymer is low and there may be a problem in economic efficiency. In contrast, when the concentration is excessively high, it may cause problems in processes in that some of the monomer may be extracted or the pulverization efficiency of the polymerized hydrogel polymer may be lowered in the pulverization process, and thus physical properties of the super absorbent polymer may be deteriorated.

In addition, the terminology 'internal cross-linking agent' used herein is different from a surface cross-linking agent for cross-linking the surface of the super absorbent polymer particles to be described later, and the internal cross-linking agent polymerizes unsaturated bonds of the water-soluble ethylene-based unsaturated monomers by cross-linking. The cross-linking in the above step proceeds both on the surface and on the inside, but when the surface cross-linking process of the super absorbent polymer particles to be described later is in progress, the surface of the particles of the finally prepared super absorbent polymer has a structure cross-linked by a surface cross-linking agent, and the inside of the particles has a structure cross-linked by the internal cross-linking agent.

As the internal cross-linking agent, any compound may be used as long as it allows the introduction of cross-linking bonds during polymerization of the acrylic acid-based monomer. Specifically, the internal cross-linking agent may be a cross-linking agent having one or more ethylene-based unsaturated groups in addition to the functional group which may react with the water-soluble substituents of the acrylic acid-based monomer; or a cross-linking agent having two or more functional groups which may react with the water-soluble substituents of the monomer and/or the water-soluble substituents formed by hydrolysis of the monomer.

As the internal cross-linking agent, any compound may be used as long as it allows the introduction of cross-linking bonds during polymerization of the acrylic acid-based monomer. Specifically, the internal cross-linking agent may be a cross-linking agent having one or more ethylene-based unsaturated groups in addition to the functional group which may react with the water-soluble substituents of the acrylic acid-based monomer; or a cross-linking agent having two or more functional groups which may react with the water-soluble substituents of the monomer and/or the water-soluble substituents formed by hydrolysis of the monomer.

The internal cross-linking agent may be an epoxy compound or a polyethylene glycol-based polymer. For example, as the internal cross-linking agent, a multifunctional cross-linking agent may be used alone or in combination of two or more. Examples of the internal cross-linking agent include an acrylate-based compound such as N,N'-methylenebisacrylamide, trimethylpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, and pentaerythritol tetraacrylate; an epoxy-based compound such as ethylene glycol diglycidyl ether, diethylene glycol diglycidyl ether, polyethylene glycol diglycidyl ether, propylene glycol diglycidyl ether, tripropylene glycol diglycidyl ether, polypropylene glycol diglycidyl ether, neopentyl glycol diglycidyl ether, 1,6-hexanediol diglycidyl ether, polytetramethylene glycol diglycidyl ether, glycerol diglycidyl ether, glycerol triglycidyl ether, diglycerol polyglycidyl ether and polyglycerol polyglycidyl ether; triarylamine; propylene glycol; glycerin; and ethylene carbonate, but the present disclosure is not limited thereto.

According to one embodiment, the epoxy-based compound may be used as the internal cross-linking agent. For example, the internal cross-linking agent may be a divalent or higher polyvalent epoxy compound, for example, ethylene glycol diglycidyl ether. In this case, foaming by the foaming agent can be stably achieved due to the hydrophobic particles.

In the monomer composition, the internal cross-linking agent may be used in an amount of 0.01 to 5 parts by weight based on 100 parts by weight of the acrylic acid-based monomer. For example, the internal cross-linking agent may be used in an amount of 0.01 parts by weight or more, 0.05 parts by weight or more, 0.1 parts by weight, or 0.15 parts by weight or more, and 5 parts by weight or less, 3 parts by weight or less, 2 parts by weight or less, 1 parts by weight or less, or 0.7 parts by weight or less based on 100 parts by weight of the water-soluble ethylene-based unsaturated monomer. When too little internal cross-linking agent is used, cross-linking does not occur sufficiently, and thus it may be difficult to achieve strength above an appropriate level, and when too much internal cross-linking agent is used, the internal cross-linking density increases, and thus it may be difficult to achieve a desired level of centrifugal retention capacity.

The cross-linking polymerization of the water-soluble ethylene-based unsaturated monomer in the presence of an internal cross-linking agent may be performed by thermal polymerization, photopolymerization or hybrid polymerization in the presence of a polymerization initiator with or without a thickener, a plasticizer, a preservation stabilizer, an antioxidant, etc., but the specific details will be described later.

Meanwhile, the super absorbent polymer further includes a surface cross-linked layer formed by further cross-linking the cross-linked polymer in the base resin using a surface cross-linking agent on at least a part of the surface of the base resin. This is to increase the surface cross-linking density of the super absorbent polymer. When the super absorbent polymer further includes a surface cross-linked layer, it has a structure having higher cross-linking density on the outside than inside.

As the surface cross-linking agent, any surface cross-linking agent that has been conventionally used in the preparation of a super absorbent polymer may be used without any particular limitation. For example, at least one selected from the group consisting of a polyalcohol-based compound, a polyepoxy-based compound, a polyamine compound, a haloepoxy compound, a condensation product of a haloepoxy compound, an oxazoline-based compound, and an alkylene carbonate-based compound may be used.

Specifically, as the polyalcohol-based compound, mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, or 1,2-cyclohexane dimethanol may be used.

In addition, as the polyepoxy-based compound, ethylene glycol diglycidyl ether, or glycidol may be used.

As the polyamine compound, ethylenediamine, diethylenetriamine, triethylenetetraamine, tetraethylenepentamine, pentaethylenehexamine, polyethyleneimine or polyamide polyamine may be used.

Further, as the haloepoxy compound, epichlorohydrin, epibromohydrin, or $\alpha$-methylepichlorohydrin may be used.

Meanwhile, as the oxazoline-based compound, mono-, di-, or polyoxazolidinone may be used.

In addition, as the alkylene carbonate-based compound, ethylene carbonate, propylene carbonate, or glycerol carbonate may be used.

More specifically, as the surface cross-linking agent, the surface cross-linking agents described above may be used alone or in combination with each other. For example, an alkylene carbonate compound such as ethylene carbonate may be used as the surface cross-linking agent.

Meanwhile, the super absorbent polymer may contain 10 wt% or less of particles having a particle diameter of 710 $\mu$m or more and 850 $\mu$m or less based on the super absorbent polymer. Additionally, the super absorbent polymer may contain 10 wt% or less of particles having a particle diameter of 150 $\mu$m or less. The particle diameter of these super absorbent polymer particles can be measured according to the EDANA WSP 220.3 (European Disposables and Nonwovens Association, EDANA).

When the content of particles having a particle diameter of 710 $\mu$m or more and 850 $\mu$m or less is high, exceeding 10 wt%, generally thin pulpless diapers may feel hard, making them less comfortable to wear, and the absorption rate may also be reduced. In addition, when the content of particles having a particle diameter of 150 $\mu$m or less exceeds 10 wt%, a problem of filter clogging may occur during the process due to a large amount of fine powder, and there is a disadvantage in that working environment becomes uncomfortable.

Meanwhile, a rewet value measured for the pulpless absorbent body containing the super absorbent polymer may be 1 g or less. Preferably, the rewet value may be 0.1 g or more, 0.3 g or more, 0.5 g or more, or 0.7 g or more, and 1 g or less, 0.95 g or less, or 0.94 g or less.

The measurement of the rewet value can be performed by preparing a pulpless absorbent body containing a super absorbent polymer and measuring the rewet value of the absorbent body. The specific preparation method of the pulpless absorbent body and measurement method are as follows.

After uniformly applying 0.3 g of an adhesive (product name: FLC7228AZP, manufacturer: HB Fuller) (adhesive layer) on a first nonwoven fabric (product name: Softhann®, manufacturer: Sambo) of 350 mm * 100 mm in size with a hot melt sprayer, 4.5 g of the super absorbent polymer is uniformly applied thereto at a feed rate of 2 g/s to prepare a first application layer of the super absorbent polymer. 0.3 g of the adhesive is applied thereon in the same manner to prepare a first application layer of the adhesive. Thereafter, the process of preparing the first application layer of the super absorbent polymer and the first application layer of the adhesive is repeated two more time to produce a second application layer of the super absorbent polymer, a second application layer of the adhesive, a third application layer of the super absorbent polymer, and an adhesive layer. Then, an absorbent body is manufactured by attaching a second nonwoven fabric (product name: Softhann®, manufacturer: Sambo). The amount of adhesive used in each adhesive layer is the same as 0.3 g, and the amount of super absorbent polymer used in each super absorbent polymer layer is the same as 4.5 g.

Then, 85 mL of a 0.9 wt% sodium chloride aqueous solution (physiological saline) is injected into the center of an absorbent body (350 mm * 100 mm * 3 mm in size). 15 minutes later, a weight is placed on the absorbent body and an additional 85 mL of physiological saline is injected at the same location while applying a pressure of 0.42 psi. 15 minutes later, the weight on the absorbent body is temporarily removed and a new paper (filter paper of 300 gsm (S-300, Hankuk Paper), approximately 1.5 g($W_5$(g))/sheet) is placed on the absorbent body. Then, the weight is placed back on the paper to position the paper between the absorbent body and the weight. 2 minutes later, the amount of physiological saline emerged from the absorbent body to the paper is measured, and the rewet (g) is calculated according

to the following Equation 3.

[Equation 3]

$$\text{Rewet (g)} = W_6\text{(g)} - W_5\text{(g)}$$

In Equation 3,

$W_5$ (g) is an initial weight of the paper, and $W_6$ (g) is a weight of the paper that absorbed the liquid emerged from the absorbent body for 2 minutes under a load (0.42 psi) after injecting physiological saline into the absorbent body under no pressure and under pressure.

**The preparation method of super absorbent polymer**

The super absorbent polymer can be prepared by a preparation method including a step of forming a hydrogel polymer by polymerizing a monomer composition containing an acrylic acid-based monomer having at least partially neutralized acidic groups, an internal cross-linking agent, an initiator, and hydrophobic particles (step 1); a step of forming a base resin powder by drying the hydrogel polymer (step 2); a first pulverization step of pulverizing the base resin powder to include 50 wt% or more of particles with a particle diameter of 710 $\mu$m or more (step 3); a second pulverization step of pulverizing the base resin powder obtained after the first pulverization step to include 10 wt% or less of particles with a particle diameter of 710 $\mu$m or more and 10 wt% or less of particles with a particle diameter of 150 $\mu$m or less (step 4); and a step of cross-linking the surface of the base resin in the presence of a surface cross-linking agent (step 5). Herein, the super absorbent polymer satisfies the above-mentioned centrifugal retention capacity, extractable content measured after swelling for 16 hours, absorption rate, and bulk density.

Hereinafter, the preparation method of the super absorbent polymer will be described in more detail step by step according to specific embodiments of the present invention.

**(Step 1)**

In the preparation method of one embodiment, the step 1 is a step of preparing a monomer composition containing an acrylic acid-based monomer having at least partially neutralized acidic groups, and an internal cross-linking agent, and forming a hydrogel polymer by cross-linking polymerization of the monomer composition in the presence of hydrophobic particles. For details on the acrylic acid-based monomer and internal cross-linking agent, refer to the above.

In the monomer composition, the internal cross-linking agent may be used in an amount of 0.01 to 5 parts by weight based on 100 parts by weight of the acrylic acid-based monomer. For example, the internal cross-linking agent may be used in an amount of 0.01 parts by weight or more, 0.05 parts by weight or more, 0.1 parts by weight, or 0.45 parts by weight or more, and 5 parts by weight or less, 3 parts by weight or less, 2 parts by weight or less, 1 parts by weight or less, or 0.7 parts by weight or less based on 100 parts by weight of the acrylic acid-based monomer. When too little internal cross-linking agent is used, cross-linking does not occur sufficiently, and thus it may be difficult to achieve strength above an appropriate level, and when too much internal cross-linking agent is used, the internal cross-linking density increases, and thus it may be difficult to achieve a desired level of centrifugal retention capacity.

Additionally, the monomer composition may further include a polymerization initiator to initiate the polymerization reaction of the monomer. The polymerization initiator is not particularly limited as long as it is commonly used in the preparation of super absorbent polymers.

Specifically, the polymerization initiator may be a thermal polymerization initiator or a photopolymerization initiator by UV irradiation depending on the polymerization method. However, even in the case of performing the photopolymerization method, a certain amount of heat is generated by light irradiation such as ultraviolet irradiation. Further, a certain amount of heat may be generated with the progress of the polymerization reaction, which is an exothermic reaction, a thermal polymerization initiator can be further used.

As the photopolymerization initiator, a compound capable of forming radicals by a light such as UV can be used without limitations in the constitution.

For example, the photopolymerization initiator may be one or more compounds selected from the group consisting of benzoin ether, dialkyl acetophenone, hydroxyl alkylketone, phenyl glyoxylate, benzyl dimethyl ketal, acyl phosphine, and $\alpha$-aminoketone. Meanwhile, as the specific example of acyl phosphine, commercial lucirin TPO (diphenyl(2,4,6-trimethylbenzoyl)phosphine oxide) may be used. More various photopolymerization initiators are well disclosed in "UV Coatings: Basics, Recent Developments and New Application (Elsevier, 2007)" written by Reinhold Schwalm, p 115, and the present disclosure is not limited thereto.

The photopolymerization initiator may be contained at a concentration of about 0.01 to about 1.0 wt% based on the monomer composition. When the concentration of the photopolymerization initiator is too low, the polymerization rate may become slow, and when the concentration of the photopolymerization initiator is too high, the molecular weight of the super absorbent polymer may become low and properties may be uneven.

In addition, one or more initiators selected from the group consisting of a persulfate-based initiator, an azo-based initiator, hydrogen peroxide, and ascorbic acid may be used as the thermal polymerization initiator. Specifically, sodium persulfate ($Na_2S_2O_8$), potassium persulfate ($K_2S_2O_8$), ammonium persulfate ($(NH_4)_2S_2O_8$), and the like may be used as examples of the persulfate-based initiators; and 2,2-azobis(2-amidinopropane) dihydrochloride, 2,2-azobis-(N,N-dimethylene)isobutyramidine dihydrochloride, 2-(carbamoylazo)isobutylonitril, 2,2-azobis[2-(2-imidazolin-2-yl)propane] dihydrochloride, 4,4-azobis-(4-cyanovaleric acid), and the like may be used as examples of the azo-based initiators. More various thermal polymerization initiators are well disclosed in 'Principle of Polymerization (Wiley, 1981)' written by Odian, p 203, and the present disclosure is not limited thereto.

The thermal polymerization initiator may be included in an amount of 0.001 to 0.2 parts by weight based on 100 parts by weight of the acrylic acid-based monomer. More preferably, it may be included in an amount of 0.19 parts by weight or less, 0.18 parts by weight or less, 0.17 parts by weight or less, or 0.16 parts by weight or less. When the concentration of the thermal polymerization initiator is too low, additional thermal polymerization rarely occurs, so the effect of adding the thermal polymerization initiator may be insignificant, and the extractable content may increase, thereby lowering the absorbency under pressure (AUP). When the concentration of the thermal polymerization initiator is too high, the molecular weight of the super absorbent polymer may become small and absorption capacity and absorbency under pressure (AUP) may decrease.

The polymerization initiator may be used in an amount of 1 parts by weight or less based on 100 parts by weight of the acrylic acid-based monomer. That is, when the concentration of the polymerization initiator is too low, the polymerization rate may become slow and thus a large amount of residual monomer may be extracted from the final product, which is not preferable. On the contrary, when the concentration of the polymerization initiator is too high, a polymer chain forming a network may become short, and thus, the physical properties of polymer may be degraded, such as increase in the content of water-soluble components and decrease in absorbency under pressure, which is not preferable.

The monomer composition may further include additives such as a thickener, a plasticizer, a storage stabilizer, and an antioxidant, if necessary.

Further, the monomer composition including the monomer may be, for example, in the form of a solution dissolved in a solvent such as water, and the solid content in the monomer composition in the form of a solution, that is, the concentration of the monomer, internal cross-linking agent, and polymerization initiator can be appropriately adjusted in consideration of the polymerization time and reaction conditions. For example, the solid content in the monomer composition may be 10 to 80 wt%, 15 to 60 wt%, or 30 to 50 wt%.

When the monomer composition has a solid content within the above range, the gel effect phenomenon that appears in the polymerization reaction of a high concentration aqueous solution is used to eliminate the need to remove unreacted monomers after polymerization, and it may be advantageous to control the pulverization efficiency when pulverizing the polymer, which will be described later.

At this time, any solvent which can dissolve the above components may be used without limitation. For example, the solvent may be in combination of at least one selected from water, ethanol, ethyleneglycol, diethyleneglycol, triethyleneglycol, 1,4-butanediol, propyleneglycol, ethyleneglycol monobutylether, propyleneglycol monomethylether, propyleneglycol monomethylether acetate, methylethylketone, acetone, methylamylketone, cyclohexanone, cyclopentanone, diethyleneglycol monomethylether, diethyleneglycol ethylether, toluene, xylene, butyrolactone, carbitol, methylcellosolve acetate, and N,N-dimethylacetamide.

In addition, in Step 1, a step of preparing a hydrogel polymer by cross-linking polymerization of the monomer composition in the presence of hydrophobic particles is performed. The hydrophobic particles may exist in the form of an aqueous dispersion, and a carbonate-based foaming agent may be additionally present in the hydrogel polymer preparation step. If a carbonate-based foaming agent is present, carbon dioxide bubbles are generated from the carbonate-based foaming agent in the above step, and the hydrophobic particles dispersed in water effectively capture these bubbles, thereby increasing the specific surface area of the prepared hydrogel polymer.

Meanwhile, a bubble generator may be used in place of the foaming agent, and any microbubble generator, or the like previously used for foaming the monomer composition in the preparation of the super absorbent polymer may be used without any particular limitation. An example of such a microbubble generator passes the monomer composition through a tubular flow path having a plurality of protruding pins mounted therein at a predetermined feeding rate, for example, at 50 to 1500 (L/min), and causes the monomer composition to collide with the protruding pins to foam. The example of such a microbubble generator is disclosed in Korean Patent Publication No. 2020-0128969, and it is also possible to obtain and apply commercial products applied in the following examples.

Herein, hydrophobic particles refer to particles having a water contact angle of 50° or more or water-insoluble particles that do not dissolve in water. Particles with a water contact angle of less than 50° and water-soluble particles

may be dissolved in a monomer composition in the form of an aqueous solution, making it difficult to capture bubbles generated during the polymerization process. On the other hand, hydrophobic particles are located at the interface between the neutralizing solution and hydrophobic bubbles such as carbon dioxide in the neutralizing solution, and can effectively capture and stabilize the bubbles.

Accordingly, the hydrophobic particles have a water contact angle of 50° or more. More specifically, the hydrophobic particles may have a water contact angle of 70° or more, 100° or more, 120° or more, or 130° or more, and 175° or less.

At this time, the contact angle of the hydrophobic particles can be measured by the following method. First, a coating solution in which the hydrophobic particles are dispersed in a methylene chloride solvent at a concentration of 5 wt% is prepared. Next, this coating solution is spin-coated on a wafer without surface roughness and then dried at room temperature to remove the remaining solvent. Afterwards, water is dropped dropwise on this coating layer to measure the contact angle, which is defined as the contact angle of each hydrophobic particle.

In addition, the hydrophobic particles have an average particle diameter of 0.2 $\mu$m to 50 $\mu$m. If the average particle diameter of the hydrophobic particles is less than 0.2 $\mu$m, it is difficult to effectively collect bubbles generated during the manufacturing process, and there is a problem in that uniform pores are not formed. If the hydrophobic particles have an average particle diameter of more than 50 $\mu$m, the resulting pore size may become too large, making it difficult to improve the absorption rate of the super absorbent polymer. Specifically, the hydrophobic particles may have an average particle diameter ($\mu$m) of 0.3 or more, 0.5 or more, 1 or more, 2 or more, or 3 or more, and 40 or less, 35 or less, or 30 or less.

Herein, the average particle diameter of the hydrophobic particles means D50, and the particle diameter "Dn" means a particle diameter at the n% point of the cumulative distribution of the number of particles according to particle diameters. In other words, D50 is a particle diameter at the 50% point of the cumulative distribution of the number of particles according to particle diameters, D90 is a particle diameter at the 90% point of the cumulative distribution of the number of particles according to particle diameters, and D10 is a particle diameter at the 10% point of the cumulative distribution of the number of particles according to particle diameters. The Dn may be measured using a laser diffraction method. Specifically, the powder to be measured is dispersed in the dispersion medium and introduced into a commercially available particle size measuring device (e.g., Microtrac S3500). Then, a particle size distribution is obtained by measuring a difference in diffraction patterns according to particle diameters when the particles pass through the laser beam. In the measuring device, D10, D50 and D90 can be obtained by calculating a particle diameter at a point of reaching 10%, 50% and 90% of the cumulative distribution of the number of particles according to particle diameters.

The hydrophobic particles may be at least one selected from the group consisting of hydrophobic silica, a metal salt of a C7 to C24 fatty acid, and hydrophobic organic particles.

Herein, the hydrophobic silica is a generic term for silica having a water contact angle of 50° or more due to a small content of silanol (-SiOH) on its surface, and hydrophobic silica known in the art may be used without limitation.

In addition, the metal salt of a C7 to C24 fatty acid refers to a compound in which a metal cation is bonded instead of a hydrogen ion of a carboxyl group at the end of an unsaturated or saturated fatty acid having a linear structure while having 7 to 24 carbon atoms in the molecule, and may be a monovalent metal salt, or a polyvalent metal salt of divalent or higher. At this time, when the hydrophobic particles are a metal salt of a fatty acid having less than 7 carbon atoms, it is not possible to capture the bubbles generated in the form of particles by ionization in an aqueous solution. When the hydrophobic particles are a metal salt of a fatty acid having more than 24 carbon atoms, the chain of the fatty acid becomes long, which may cause difficult dispersion.

Specifically, when the metal salt of the fatty acid is a monovalent metal salt, it has a structure in which one fatty acid carboxylate anion is bonded to an alkali ion, which is a monovalent metal cation. In addition, when the metal salt of the fatty acid is a polyvalent metal salt of divalent or higher, it has a structure in which as many as fatty acid carboxylate anions as the number of the valence of the metal cation are bonded to the metal cation.

In one embodiment, the hydrophobic particles may be a metal salt of a C12 to C20 saturated fatty acid. For example, the hydrophobic particles may be at least one metal salt of a saturated fatty acid selected from the group consisting of a metal salt of lauric acid containing 12 carbon atoms in the molecule; a metal salt of tridecyl acid containing 13 carbon atoms in the molecule; a metal salt of myristic acid containing 14 carbon atoms in the molecule; a metal salt of pentadecanoic acid containing 15 carbon atoms in the molecule; a metal salt of palmitic acid containing 16 carbon atoms in the molecule; a metal salt of margaric acid containing 17 carbon atoms in the molecule; a metal salt of stearic acid containing 18 carbon atoms in the molecule; a metal salt of nonadecylic acid containing 19 carbon atoms in the molecule; and a metal salt of arachidic acid containing 20 carbon atoms in the molecule.

Preferably, the metal salt of a fatty acid may be a metal salt of stearic acid. For example, it may be at least one metal salt of stearic acid selected from the group consisting of calcium stearate, magnesium stearate, sodium stearate, zinc stearate and potassium stearate.

In addition, the hydrophobic organic particles may be at least one selected from the group consisting of an ethylene polymer, a propylene polymer, a styrene polymer, a butadiene polymer, a styrene-butadiene copolymer, an

alkyl acrylate polymer, an alkyl methacrylate polymer, an alkyl acrylate-acrylonitrile copolymer, an acrylonitrile-butadiene copolymer, an acrylonitrile-butadiene-styrene copolymer, an acrylonitrile-alkyl acrylate-styrene copolymer, an alkyl methacrylate-butadiene-styrene copolymer, and an alkyl acrylate-alkyl methacrylate copolymer.

In addition, the hydrophobic particles may be included in the aqueous dispersion in an amount of 10 to 70 wt% based on a total weight of the aqueous dispersion. When the content of the hydrophobic particles in the hydrophobic aqueous dispersion is too low or too high, dispersion stabilization of the hydrophobic particles cannot be achieved, and a problem of agglomeration between particles or sinkage by gravity may occur.

In addition, as the surfactant for dispersing the hydrophobic particles in the aqueous dispersion of hydrophobic particles, a surfactant known in the art that can stabilize the dispersion of the hydrophobic particles may be used without limitation. For example, one or more surfactants selected from the group consisting of cationic surfactants, anionic surfactants, amphoteric surfactants, and nonionic surfactants may be used as the surfactant. Preferably, two or more surfactants may be used for dispersion stabilization of the hydrophobic particles. More specifically, in consideration of the form of the hydrophobic particles, for example, the form of a metal salt of a saturated fatty acid, a nonionic surfactant and an anionic surfactant may be used together in order to disperse the hydrophobic particles more effectively in water. For example, a nonionic surfactant to which a long-chain hydrocarbon having 10 or more carbon atoms is bonded and a sulfate-based anionic surfactant may be used together.

For example, examples of the cationic surfactant include dialkyldimethylammonium salt and alkylbenzylmethylammonium salt, examples of the anionic surfactant include alkylpolyoxyethylene sulfate, monoalkyl sulfate, alkylbenzene sulfonate, monoalkyl phosphate, a sulfate having a functional group containing a long-chain hydrocarbon or a sodium salt thereof such as sodium lauryl sulfate, sodium dodecyl sulfate, or sodium laureth sulfate, examples of the amphoteric surfactant include alkylsulfobetaine and alkylcarboxybetaine, and examples of the nonionic surfactant include polyoxyethylene alkyl ether such as polyethylene glycol, polyoxyalkylene alkylphenyl ether, polyoxyethylene arylphenyl ether, fatty acid ester such as sorbitan monopalmitate, fatty acid sorbitan ester, or glycerin monostearate, alkyl monoglyceryl ether, alkanolamide, and alkyl polyglycoside. However, the present disclosure is not limited thereto.

In addition, the aqueous dispersion of hydrophobic particles may have a pH of 7 or more. When the pH of the aqueous dispersion of hydrophobic particles is less than 7, it is acidic, so it is difficult to stabilize the hydrophobic particles, which are metal salts of fatty acids, which is not suitable.

Meanwhile, the hydrophobic particles may be used in an amount of 0.01 to 0.5 parts by weight based on 100 parts by weight of the acrylic acid-based monomer. When the content of the hydrophobic particles is too low, the bubble stabilizing effect may not be sufficient, and thus the absorption rate may be slowed. When the content of the hydrophobic particles is too high, the amount of the surfactant used to stabilize the hydrophobic particles in the aqueous dispersion of the hydrophobic particles increases, so that the surface tension may be lowered. For example, the hydrophobic particles may be used in an amount of 0.01 parts by weight or more, 0.03 parts by weight or more, 0.05 parts by weight or more, or 0.08 parts by weight or more, and 0.5 parts by weight or less, 0.4 parts by weight or less, 0.3 parts by weight or less, or 0.2 parts by weight or less based on 100 parts by weight of the acrylic acid-based monomer.

In addition, the carbonate-based foaming agent serves to increase the surface area by foaming during polymerization to form pores in the hydrogel polymer. For example, it may be at least one selected from the group consisting of sodium bicarbonate, sodium carbonate, potassium bicarbonate, potassium carbonate, calcium bicarbonate, calcium carbonate, magnesium bicarbonate and magnesium carbonate.

The carbonate-based foaming agent may be used in an amount of 0.005 to 1 part by weight based on 100 parts by weight of the acrylic acid-based monomer. When the content of the foaming agent is less than 0.005 parts by weight, the effect of using the foaming agent may be insignificant. When the content of the foaming agent exceeds 1 part by weight, there are too many pores in the cross-linked polymer, so that the gel strength of the super absorbent polymer to be prepared decreases and the density decreases, which may cause problems in distribution and storage. For example, the carbonate-based foaming agent may be used in an amount of 0.01 parts by weight or more, or 0.05 parts by weight or more, and 0.5 parts by weight or less, 0.3 parts by weight or less, or 0.2 parts by weight or less based on 100 parts by weight of the acrylic acid-based monomer.

In addition, the carbonate-based foaming agent and the hydrophobic particles may be used in a weight ratio of 1:0.1 to 1:2. When the hydrophobic particles are used in an excessively low content compared to the carbonate-based foaming agent, it is difficult to effectively capture the generated bubbles. When they are used in an excessively high content compared to the foaming agent, various physical properties such as centrifugal retention capacity and absorption rate may decrease. Specifically, the carbonate-based foaming agent and the hydrophobic particles may be used in a weight ratio of 1:0.4 or more, 1:0.6 or more, or 1:0.8 or more, and 1:1.7 or less, 1:1.5 or less, or 1:1.2 or less. For example, the carbonate-based foaming agent and the hydrophobic particles may be used in a weight ratio of 1:1.

Additionally, surfactants such as alkyl sulfate-based compounds and polyoxyethylene alkyl ether-based compounds that are commonly used as s foam stabilizer may not be used in Step 1. For example, in the steps 1 and 2, cationic surfactants such as quaternary ammonium compounds e.g., dodecyltrimethylammonium chloride or dodecyltrimethylammonium bromide; anionic surfactants such as alkyl sulfate-based compounds, e.g., sodium dodecyl sulfate,

ammonium lauryl sulfate, sodium lauryl ether sulfate, or sodium myreth sulfate; or nonionic surfactants such as alkyl ether sulfate-based compounds, e.g., polyoxyethylene lauryl ether may not be used. Accordingly, the problem of lowering the surface tension of the super absorbent polymer caused by the use of the surfactant can be prevented.

Meanwhile, the polymerization of the monomer composition in the presence of such an aqueous dispersion of hydrophobic particles and a carbonate-based foaming agent can proceed without particular limitation, as long as it is a commonly used polymerization method.

Specifically, the polymerization method is largely divided into the thermal polymerization and the photopolymerization according to an energy source of the polymerization. In the case of thermal polymerization, it is generally carried out in a reactor equipped with an agitation spindle, such as a kneader. In the case of photopolymerization, it may be carried out in a reactor equipped with a movable conveyor belt. However, the polymerization method is just an example, and the present disclosure is not limited thereto.

For example, in the reactor equipped with an agitation spindle such as a kneader, the hydrogel polymer obtained by thermal polymerization by supplying hot air or heating the reactor may be discharged to a reactor outlet in the form of several centimeters to several millimeters depending on a shape of the agitation spindle provided in the reactor. Specifically, a size of the hydrogel polymer obtained may vary depending on the concentration and injection rate of the monomer mixture to be injected, and a hydrogel polymer having a weight average particle diameter of 2 to 50 mm may be usually obtained.

In addition, when photopolymerization is performed in the reactor equipped with a movable conveyor belt as described above, a hydrogel polymer in the form of a sheet having a belt width may usually be obtained. At this time, a thickness of the polymer sheet may vary depending on the concentration and injection rate of the monomer composition to be injected, and it is preferable to supply the monomer composition so that the polymer in the form of a sheet has a thickness of 0.5 to 5 cm. When the monomer composition is supplied to such an extent that the thickness of the polymer sheet is too thin, the production efficiency may be low. When the thickness of the polymer sheet exceeds 5 cm, the polymerization reaction may not occur evenly over the entire thickness due to the excessively thick thickness.

In this case, the hydrogel polymer obtained by the above method may have a moisture content of about 40 to about 80 wt%. At this time, "moisture content" in the present disclosure is the content of moisture in the entire weight of the polymer, and it means a value of which the weight of the dried polymer is subtracted from the weight of the polymer. Specifically, the moisture content is defined as a value calculated by measuring the weight loss due to moisture evaporation from the polymer in the process of increasing the temperature of the polymer and drying the same through infrared heating. At this time, the drying condition for measuring the moisture content is as follows: the temperature is increased to about 180 °C and maintained at 180 °C, and the total drying time is 40 minutes including 5 minutes of a heating step.

**(Step 2)**

Subsequently, a step of drying the hydrogel polymer to form a base resin in the form of powder is performed. If necessary, a coarse pulverization step may be further performed before drying to increase the efficiency of the drying step.

The coarse pulverization step is a step for increasing drying efficiency in the subsequent drying step and controlling the particle size of the super absorbent polymer powder to be prepared. At this time, a pulverizing machine used herein may include, but its configuration is not limited to, any one selected from the group consisting of a vertical pulverizer, a turbo cutter, a turbo grinder, a rotary cutter mill, a cutter mill, a disc mill, a shred crusher, a crusher, a meat chopper, and a disc cutter. However, the present disclosure is not limited thereto.

The gel pulverization of the hydrogel polymer may be performed so that the particle size of the hydrogel polymer is 0.01 mm to 50 mm, or 0.01 mm to 30 mm. That is, in order to increase the drying efficiency, it is preferable that the hydrogel polymer is pulverized into particles of 50 mm or less. However, since excessive pulverization may cause agglomeration between particles, it is preferable that the hydrogel polymer is gel-pulverized into particles of 0.01 mm or more.

In addition, since the gel pulverization of the hydrogel polymer is performed in a state where the moisture content is relatively low, a phenomenon in which the hydrogel polymer adheres to the surface of the gel pulverizing machine may occur. In order to minimize this phenomenon, if necessary, steam, water, a surfactant, an anti-agglomeration agent (e.g., clay, silica, etc.), a persulfate-based initiator, an azo-based initiator, hydrogen peroxide, a thermal polymerization initiator, an epoxy-based cross-linking agent, a diol-based cross-linking agent, a cross-linking agent containing a polyfunctional acrylate of difunctional, trifunctional or higher, a monofunctional cross-linking agent containing a hydroxyl group, or the like may be added to the hydrogel polymer.

Drying is performed on the hydrogel polymer that has been chopped as described above or immediately after polymerization without the chopping and pulverization step. The drying temperature in the drying step may be about 150 to about 250 °C. When the drying temperature is less than 150 °C, the drying time may become excessively long

and physical properties of the super absorbent polymer to be finally formed may decrease. When the drying temperature is more than 250 °C, only the surface of the polymer is excessively dried, fine powder may be generated in the subsequent pulverization process, and physical properties of the final super absorbent polymer may decrease. Therefore, the drying may preferably be performed at a temperature of about 150 to about 200 °C, more preferably at a temperature of about 160 to about 180 °C.

Meanwhile, the drying may be performed for about 20 to about 90 minutes, in consideration of the process efficiency and the like, but it is not limited thereto.

The drying method in the drying step is not particularly limited if it has been generally used in the drying process of the hydrogel polymer. Specifically, the drying step may be performed by the method of hot air provision, infrared radiation, microwave radiation, UV ray radiation, and the like. After the drying step, the moisture content of the polymer may be about 0.1 to about 5 wt%.

**(Step 3 & Step 4)**

Subsequently, a first pulverization step (step 3) and a second pulverization step (step 4) in which the base resin powder is pulverized according to the particle size distribution are performed. If necessary, a drying step may be included before performing step 3 or before performing step 4.

Specifically, in step 3, the base resin powder is pulverized (first pulverization step) to include 50 wt% or more of particles with a particle diameter of 710 $\mu$m or more (first pulverization step). In step 4, the base resin powder obtained after the first pulverization step is pulverized to include 10 wt% or less of particles with a particle diameter of 710 $\mu$m or more and 10 wt% or less of particles with a particle diameter of 150 $\mu$m or less (second pulverization step).

Specifically, 50 wt% or more of particles with a particle diameter of 710 $\mu$m or more are included through the classification process after the first pulverization step, regardless of the type of pulverizing machine. Particles with a particle diameter of 710 $\mu$m or more are subjected a separate second pulverization process, and the base resin powder obtained after the first and second pulverization contains 10 wt% or less of particles with a particle diameter of 710 $\mu$m or more and 850 $\mu$m or less, and 10 wt% or less of particles with a particle diameter of 150 $\mu$m or less.

The pulverization conditions can be adjusted such that 50 wt% or more of particles with a particle diameter of 710 $\mu$m or more are included after the first pulverization. When using a coarse pulverizing machine, the degree of pulverization can be adjusted by rpm of the pulverizing machine, and when using a roll mill, the first pulverization can be performed by adjusting the spacing of the roll mill. The polymer obtained after the first pulverization is subjected to a classification step, and a classification mesh can be constructed to separate particles with a particle diameter of 710 $\mu$m or more through the classification process. Particles with a particle diameter of 710 $\mu$m or more is subjected to a second pulverization process. In the second pulverization process, the type of pulverizing machine is not particularly limited as in the first pulverization process. The polymer obtained after the second pulverization can be included in the base resin with or without a classification process, and the final base resin powder should contain 10 wt% or less of particles with a particle diameter of 710 $\mu$m or more and 850 $\mu$m or less, and 10 wt% or less of particles with a particle diameter of 150 $\mu$m or less.

When the final base resin powder contains 10 wt% or less of particles with a particle diameter of 710 $\mu$m or more and 850 $\mu$m or less, and 10 wt% or less of particles with a particle diameter of 150 $\mu$m or less by drying a hydrogel with a fast absorption rate and performing only the first pulverization, the bulk density may be lowered.

Preferably, a bulk density of the base resin powder after the first pulverization step may be 0.50 g/ml or less.

The base resin, which is the polymer powder obtained after the pulverization step, may have a particle diameter of about 150 to about 850 $\mu$m. As the pulverizing machine used for pulverization to such a particle diameter, a pin mill, a hammer mill, a screw mill, a roll mill, a disc mill, a jog mill, or the like may be used, but the present disclosure is not limited thereto.

Further, in order to manage the physical properties of the super absorbent polymer powder to be commercialized after the pulverization step, the base resin obtained after pulverization is classified according to particle size. Preferably, the polymer having a particle diameter of about 150 to about 850 $\mu$m is classified, and only the base resin having such a particle diameter can be commercialized after a surface cross-linking reaction step. The particle diameter may be measured according to the EDANA WSP 220.3 (European Disposables and Nonwovens Association, EDANA).

**(Step 5)**

Meanwhile, after the base resin powder is prepared through the above-described classification process, the base resin powder can be surface cross-linked by heat treatment in the presence of a surface cross-linking agent to form super absorbent resin particles. The surface cross-linking is to induce a cross-linking reaction on the surface of the base resin powder in the presence of a surface cross-linking agent, and this surface cross-linking can form a surface modified layer (surface cross-linked layer) on the surface of the base resin powder.

The content of the surface cross-linking agent may be appropriately selected depending on the type of surface cross-linking agent added or reaction conditions, but may be about 0.001 to about 5 parts by weight based on 100 parts by weight of the base resin. When the content of the surface cross-linking agent is too low, surface modification may not occur properly, and physical properties of the final polymer may be deteriorated. Conversely, when an excessive amount of surface cross-linking agent is used, basic absorption performance of the polymer may be reduced due to excessive surface cross-linking reaction, which is not desirable.

The method of mixing the surface cross-linking agent with the base resin is not particularly limited. For example, a method of adding the surface cross-linking agent and the base resin powder in a reactor for mixing, a method of spraying the surface cross-linking agent onto the base resin powder, or a method of mixing the base resin and the surface cross-linking agent while continuously providing them to a continuously operating mixer may be used.

When adding the surface cross-linking agent, water may be mixed therewith and added in the form of a surface cross-linking solution. When adding water, there is an advantage in that the surface cross-linking agent can be evenly dispersed in the polymer. At this time, the content of water added is about 1 to about 10 parts by weight based on 100 parts by weight of the base resin for the purpose of inducing even dispersion of the surface cross-linking agent, preventing agglomeration of the polymer powder, and optimizing a surface penetration depth of the surface cross-linking agent.

In addition to the surface cross-linking agent, the above-described surface cross-linking step further uses at least one selected from the group consisting of polyvalent metal salts such as aluminum salts, e.g., sulfate, potassium salt, ammonium salt, sodium salt, and hydrochloride of aluminum.

As this polyvalent metal salt is additionally used, permeability of the super absorbent polymer manufactured by the method of one embodiment can be further improved. This polyvalent metal salt may be added to the surface cross-linking solution together with the surface cross-linking agent, and may be used in an amount of 0.01 to 4 parts by weight based on 100 parts by weight of the base resin powder.

Further, in order to achieve uniform surface cross-linking, the surface cross-linking solution containing the surface cross-linking agent and the liquid medium may optionally further include a surfactant, a polycarboxylic acid-based copolymer having repeating units represented by the following Chemical Formulae 1-a and 1-b, or an aliphatic alcohol having 6 or more carbon atoms. When using multiple types of surface cross-linking agents and additional components optionally included in the surface cross-linking solution, a relatively low surface tension of the surface cross-linking solution is achieved, thereby producing a super absorbent polymer having the above-described physical properties:

[Chemical Formula 1-a]

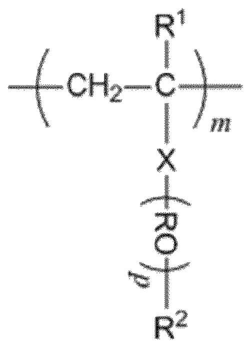

[Chemical Formula 1-b]

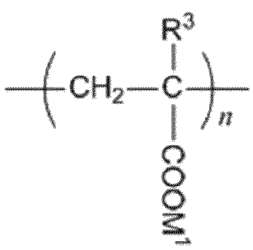

in Chemical Formulae 1-a and 1-b,
$R^1$, $R^2$ and $R^3$ are each independently hydrogen or a C1 to C6 alkyl group, RO is a C2 to C4 oxyalkylene group, $M^1$ is hydrogen, or a monovalent metal or non-metal ion, X is -COO-, a C1 to C5 alkyloxy group, or a C1 to C5 alkyldioxy group, m is an integer of 1 to 100, n is an integer of 1 to 1000, p is an integer of 1 to 150, and when p is 2 or more, two or more repeating -RO- are the same as or different from each other.

Meanwhile, the surface cross-linking process can be performed using a surface cross-linking solution containing water and/or a hydrophilic organic solvent (e.g., alcohol-based polar organic solvent such as methanol) as a liquid medium, along with the above-mentioned surface cross-linking agent. At this time, the amounts of water and a hydrophilic organic solvent to be added based on 100 parts by weight of the base resin powder may be properly controlled for the purposes of inducing a uniform dispersion of the surface cross-linking agent, preventing an agglomeration phenomenon of the base resin powder, and optimizing a surface penetration depth of the surface cross-linking agent.

The method of adding the surface cross-linking solution to the base resin powder is not particularly limited. For example, a method of adding the surface cross-linking solution and the base resin powder in a reactor for mixing, a method of spraying the surface cross-linking solution onto the base resin powder, or a method of mixing the base resin powder and the surface cross-linking solution while continuously providing them to a continuously operating mixer may be used.

Specifically, the surface cross-linking may be performed by heating the base resin powder to which the surface cross-linking solution has been added from an initial temperature of 20 °C to 130 °C to a maximum temperature of 140 °C to 200 °C over 10 to 30 minutes, and maintaining the maximum temperature for 5 to 60 minutes. More specifically, the heat treatment may be performed by maintaining the maximum temperature of 140 °C to 200 °C, or 170 °C to 195 °C for 5 to 60 minutes, or 10 to 50 minutes.

When satisfying these surface cross-linking process conditions (particularly, the temperature elevating condition and the reaction condition at maximum reaction temperature), a super absorbent polymer that appropriately satisfies physical properties of the embodiment can be more effectively produced.

The heating means for the surface cross-linking reaction is not particularly limited. It is possible to provide a thermal media thereto or provide a heat source directly thereto. At this time, usable thermal media may be a heated fluid such as steam, hot air, hot oil, and the like, but the present invention is not limited thereto. Furthermore, the temperature of the thermal media provided thereto may be properly selected in consideration of the means of the thermal media, heating speed, and target temperature of heating. Meanwhile, an electric heater or a gas heater may be used as the heat source provided directly, but the present disclosure is not limited thereto. After forming a surface cross-linked layer on the surface of the base resin as described above, inorganic materials may be additionally mixed.

The inorganic material may be, for example, at least one selected from the group consisting of silica, clay, alumina, silica-alumina composite, and titania, and preferably silica.

The inorganic material may be used in an amount of 0.01 parts by weight or more, 0.05 parts by weight or more, or 0.1 parts by weight or less, and 5 parts by weight or less, 3 parts by weight or less, or 1 part by weight or less, based on 100 parts by weight of the super absorbent polymer.

The super absorbent polymer obtained according to the above-described preparation method maintains excellent absorption performance such as centrifugal retention capacity and absorbency under pressure, and has the improved absorption rate and excellent rewet properties, thereby satisfying all physical properties of one embodiment. Accordingly, hygiene articles containing the super absorbent polymer can be provided. For example, the super absorbent polymer may be suitably used as a hygiene article such as a diaper, in particular, an ultra-thin hygiene article with a reduced pulp content.

Hereinafter, preferred embodiments are presented to facilitate the understanding of the invention. However, the following examples are for illustrative purposes only and are not intended to limit the present disclosure.

### Preparation Example 1: Preparation of aqueous dispersion of hydrophobic silica

While stirring at 5000 rpm after adding 100 g of water to a high shear mixer, hydrophobic silica with an average particle diameter of 0.3 $\mu$m and a water contact angle of 130° and hydrophobic silica with an average particle diameter of 3 $\mu$m and a water contact angle of 130° were slowly added to be dispersed in an amount of 0.2 wt% and 2 wt% based on the total weight of the final aqueous dispersion, respectively. When the silica was completely added, stirring was performed at 8000 rpm for 30 minutes at a temperature of 45 °C. At this time, the pH of the obtained aqueous dispersion of hydrophobic silica was 9.

### Example 1

**(Step 1)** A monomer solution was prepared by mixing 100 parts by weight of acrylic acid, 0.01 parts by weight of PEGDA 400 (polyethylene glycol diacrylate 400) as an internal cross-linking agent, and 0.1 part by weight of phenyl-

bis(2,4,6-trimethylbenzoyl)phosphine oxide as a photoinitiator. Subsequently, a monomer composition was prepared by continuously adding 160 parts by weight of a 24 wt% aqueous solution of sodium hydroxide while continuously supplying the monomer solution using a metering pump, and then high-speed mixing 3 parts by weight of 4 wt% aqueous solution of sodium persulfate, 5 parts by weight of 4 wt% ethylene glycol diglycidyl ether (EJ1 030s), and 5 parts by weight of 4 wt% aqueous solution of sodium bicarbonate, and 5 parts by weight of the aqueous dispersion of hydrophobic silica prepared in Preparation Example 1 containing 0.2 wt% of hydrophobic silica with an average particle diameter of 0.3 $\mu$m and 2 wt% of hydrophobic silica with an average particle diameter of 3 $\mu$m. Through this transfer, the monomer composition was added to a polymerization reactor including a moving conveyor belt, and UV polymerization was performed for 3 minutes by irradiating ultraviolet rays through a UV irradiation device to prepare a sheet-type hydrogel polymer.

**(Step 2)** The hydrogel polymer was cut to an average size of about 300 mm or less, and then placed in a pulverizing machine (equipped with a perforated plate containing a plurality of holes with a diameter of 15 mm) to pulverize the hydrogel. Thereafter, the pulverized hydrogel was dried in a dryer capable of changing wind direction up and down. The hydrogel was dried uniformly by flowing hot air at 180 °C so that the moisture content of the dried powder was about 5% or less.

**(Step 3)** The dried polymer was pulverized with a pulverizing machine and then classified to obtain a base resin with a size of 150 to 850 $\mu$m. The used pulverizing machine was PULVERISETTE 19 from FRITSCH. A 12 mm sieve cassette was installed in the pulverizing machine, and the dried hydrogel was first pulverized. Among the sieves (W.S. Tylor, 8" STAINLESS - STAINLESS TEST SIEVE) below, the sieves with mesh number of 24/32/48/100/pan were installed sequentially. Then, the pulverized particles were added to the top of the #24 mesh, fixed on a sieve shaker, and classified at 1.5 amplitude for 10 minutes.

8" STAINLESS - STAINLESS TEST SIEVES.

| Catalog Number | Description | Mesh | Microns |
|---|---|---|---|
| 040A-805-20 | 8"-FH-SS-SS-US-20 | 20 | 850 |
| 040A-805-25 | 8"-FH-SS-SS-UA-25 | 24 | 710 |
| 040A-805-35 | 8"-FH-SS-SS-US-35 | 32 | 500 |
| 040A-805-50 | 8"-FH-SS-SS-US-50 | 48 | 300 |
| 040A-805-100 | 8"-FH-SS-SS-US-100 | 100 | 150 |
| 040A-805-170 | 8"-FH-SS-SS-US-170 | 170 | 90 |
| 040A-805-325 | 8"-FH-SS-SS-US-325 | 325 | 45 |

After the classification was completed, the polymer content at the top of the #24 mesh was measured, and 50 wt% or more of particles were measured compared to the total amount of dried hydrogel added.

The particles at the top of the #24 mesh were subjected to second pulverization. In the second pulverization, a 1.0 mm sieve cassette was installed in the pulverizing machine and the dried hydrogel was pulverized. Among the sieves (W.S. Tylor, 8" STAINLESS - STAINLESS TEST SIEVE), the sieves with mesh number of 20/32/48/100/pan were installed sequentially. Then, the particles that had undergone the second pulverization were added to the top of the #20 mesh, fixed on a sieve shaker, and classified at 1.5 amplitude for 10 minutes.

The particle weight after classification can be obtained as follows:

Particle size distribution according to particle size = {(sieve weight + dried body after classification) - (empty sieve weight)} /(dried body after pulverization) *100

**(Step 4)** 8 parts by weight of an aqueous surface cross-linking solution containing 1.2 parts by weight of ethylene carbonate, 0.5 parts by weight of propylene carbonate, 0.5 parts by weight of propylene glycol, and 0.5 parts by weight of hydrophilic water-dispersed silica (snotex) was sprayed onto 100 parts by weight of the base resin powder obtained by classification, and stirred at room temperature to ensure that the surface cross-linking solution was evenly distributed on the base resin powder. Thereafter, the base resin powder mixed with the surface cross-linking solution was added to a surface cross-linking reactor and a surface cross-linking reaction was performed.

In this surface cross-linking reactor, the temperature of the base resin powder was gradually increased from an initial temperature around 80 °C, and it was operated to reach a maximum reaction temperature of 190 °C after 30

minutes. After reaching this maximum reaction temperature, the reaction was further performed for 15 minutes and a sample of the finally produced super absorbent polymer was taken. After the surface cross-linking process, the super absorbent polymer of Example 1 having a particle size of 150 μm to 850 μm was prepared by classification with a ASTM standard mesh.

**Example 2**

A super absorbent polymer was prepared in the same manner as in Example 1, except that step 4 in Example 1 was performed as follows.

A surface cross-linking solution was prepared by mixing 4 parts by weight of water, 1.0 parts by weight of propylene glycol, 0.08 parts by weight of ethylene glycol diglycidyl ether, 0.05 parts by weight of a polycarboxylic acid-based copolymer (copolymer of methoxy polyethylene glycol monomethacrylate and methacrylic acid, Mw=40,000) based on 100 parts by weight of the base resin. The surface cross-linking solution was sprayed and mixed with 100 parts by weight of the obtained base resin powder, placed in a container including a stirrer and a double jacket, and a surface cross-linking reaction was performed at 140 °C for 35 minutes. Afterwards, the surface-treated powder was classified using a ASTM standard mesh to obtain super absorbent polymer powder with a particle size of 150 to 850 μm.

**Example 3**

A super absorbent polymer was prepared in the same manner as in Example 1, except that 4 parts by weight of 4 wt% aqueous solution of sodium persulfate was used in Step 1 of Example 1.

**Example 4**

The final super absorbent polymer was prepared by blending 0.2 parts by weight of fumed silica (Aerosil 200) with the super absorbent polymer prepared in Example 3.

**Comparative Example 1**

A super absorbent polymer was prepared in the same manner as in Example 1, except that step 3 in Example 1 was performed as follows.

The dried polymer was pulverized with a pulverizing machine and then classified to obtain a base resin with a size of 150 to 850 μm. The used pulverizing machine was PULVERISETTE 19 from FRITSCH. A 0.75 mm sieve cassette was installed in the pulverizing machine, and the dried hydrogel was first pulverized. Among the sieves (W.S. Tylor, 8" STAINLESS - STAINLESS TEST SIEVE) of Example 1, the sieves with mesh number of 20/32/48/100/pan were installed sequentially. Then, the pulverized particles were added to the top of the #20 mesh, fixed on a sieve shaker, and classified at 1.5 amplitude for 10 minutes. The pulverization step was completed without a second pulverization.

**Comparative Example 2**

A super absorbent polymer was prepared in the same manner as in Example 1, except that step 3 in Example 1 was performed as follows.

The dried polymer was pulverized with a pulverizing machine and then classified to obtain a base resin with a size of 150 to 850 μm. The used pulverizing machine was PULVERISETTE 19 from FRITSCH. A 2.0 mm sieve cassette was installed in the pulverizing machine, and the dried hydrogel was first pulverized. Among the sieves (W.S. Tylor, 8" STAINLESS - STAINLESS TEST SIEVE) of Example 1, the sieves with mesh number of 20/32/48/100/pan were installed sequentially. Then, the pulverized particles were added to the top of the #20 mesh, fixed on a sieve shaker, and classified at 1.5 amplitude for 10 minutes. The pulverization step was completed without a second pulverization.

**Comparative Example 3**

A super absorbent polymer was prepared in the same manner as in Example 1, except that 0.2 parts by weight of span80 (manufactured by Sigma-Aldrich) was used instead of the hydrophobic particles.

**Comparative Example 4**

A super absorbent polymer was prepared in the same manner as in Example 1, except that gas was injected using an air bubble injector and 0.3 parts by weight of sodium dodecyl sulfate was used instead of sodium bicarbonate

and hydrophobic particles.

**Experiment Examples: Measurement of physical properties of super absorbent polymer**

The physical properties of the super absorbent polymers prepared in Examples and Comparative Examples were evaluated in the following manner and are listed in Table 1 below. Unless otherwise indicated, all procedures were conducted at room temperature (23±1 °C) and a relative humidity of 45±1%, and physiological saline or saline means a 0.9 wt% sodium chloride (NaCl) aqueous solution at 23±1 °C.

(1) Bulk density (g/ml): particles with a particle size of 150 - 850 μm were measured

About 100 g of the super absorbent polymers in all particle size distribution of Examples and Comparative Examples were placed in a funnel-shaped bulk density meter and flowed into a 100 ml container, and then the weight of the super absorbent polymers in the container was measured. The bulk density was calculated as (super absorbent polymer weight)/(container volume, 100 ml).

The measurement of bulk density was performed according to the EDANA NWSP 251.0.R2 (15).

The density cup was cylindrical and made of stainless steel (ISO/TR 15510), and had a capacity of 100.0 ± 0.5 ml, an internal diameter of 45.0 ± 0.1 mm, and an internal height of 63.1 ± 0.1 mm.

The funnel was made of stainless steel and the detailed design followed ISO/TR 15510. It had an orifice internal diameter of 10.00 ± 0.01 mm, an inclination angle of cone generatrix of 20°, a height of 145.0 ± 0.5 mm.

The analysis of bulk density was performed at 23 ± 2 °C and a relative humidity of 45 ± 15%.

First, the density cup was placed under the funnel, and 100 g of SAP was filled into the funnel closed with an orifice. A timer was started when opening the orifice, and the time until all SAP fell down the funnel was measured. SAP overflowing from the density cup was removed and its weight was measured (W2). After measuring the weight of the empty density cup (W1), the weight of SAP in the density cup could be measured by calculating the difference between the two weights.

Bulk density (ρ, g/ml) was calculated as "ρ = (W2-W1)/100", where 100 (ml) is the volume of the density cup.

(2) Extractable content measured after swelling for 16 hours (16hr E/C, %): particles with a particle size of 150 - 850 μm were measured

The extractable content of Examples and Comparative Examples was measured according to the EDANA NWSP 270.0.R2 (15).

(3) Centrifuge retention capacity (CRC, g/g): particles with a particle size of 150 - 850 μm were measured

The centrifuge retention capacity by absorption ratio under a non-loading condition of each polymer was measured according to the EDANA NWSP 241.0.R2 (15).

Specifically, after inserting WO (g, about 0.2 g) of the super absorbent polymer in all particle size distribution uniformly in a nonwoven fabric envelope and sealing the same, it was soaked in saline (0.9 wt%) at room temperature. After 30 minutes, the envelope was centrifuged at 250G for 3 minutes to drain, and the weight W2 (g) of the envelope was measured. Further, after carrying out the same operation without using the resin, the weight W1 (g) of the envelope was measured. Then, CRC (g/g) was calculated by using the obtained weight values according to the following Equation.

[Equation 1]

$$CRC (g/g) = \{[W2(g) - W1(g)]/W0(g)\} - 1$$

(4) 0.7AUP (Absorbency under pressure, g/g): particles with a particle size of 150 - 850 μm were measured

The absorbency under pressure at 0.7 psi of each polymer was measured according to the EDANA NWSP 242.0.R2 (15).

Specifically, a 400 mesh stainless steel screen was installed in a cylindrical bottom of a plastic having an inner diameter of 60 mm. WO (g, 0.90 g) of the super absorbent polymer in all particle size distribution was uniformly scattered on the screen at room temperature and a humidity of 50%. Thereafter, a piston which can uniformly provide a load of 0.7 psi was placed thereon. Herein, the outer diameter of the piston was slightly smaller than 60 mm, there was no gap with the inner wall of the cylinder, and jig-jog of the cylinder was not interrupted. At this time, the weight W3 (g) of the

device was measured.

Subsequently, a glass filter having a diameter of 90 mm and a thickness of 5 mm was placed in a petri dish having a diameter of 150 mm, and saline (0.9 wt% sodium chloride) was poured in the dish. At this time, the saline was poured until the surface level of the saline became equal to the upper surface of the glass filter. One sheet of filter paper with a diameter of 90 mm was placed thereon. After the measuring device was placed on the filter paper, the liquid was absorbed for 1 hour under a load. After 1 hour, the measuring device was lifted, and the weight W4 (g) was measured.

Then, absorbency under pressure (g/g) was calculated by using the obtained weight values according to the following Equation.

[Equation 2]

$$AUP(g/g) = [W4(g) - W3(g)]/W0(g)$$

(5) Vortex time (Absorption rate, s): particles in all particle size distribution were measured

The vortex time (absorption rate) of the super absorbent polymers of Examples and Comparative Examples was measured in the following manner.

① First, 50 mL of 0.9% saline was added to a 100 mL beaker with a flat bottom using a 100 mL Mass Cylinder.
② Next, after placing the beaker in the center of a magnetic stirrer, a cylindrical magnetic bar (diameter * length = 8 mm * 30 mm) was put in the beaker.
③ Thereafter, the stirrer was operated such that the magnetic bar stirred at 600 rpm, and the lowermost part of vortex generated by the stirring was made to reach the top of the magnetic bar.
④ After confirming that the temperature of the saline in the beaker reached 24.0 °C, 2± 0.01 g of a super absorbent polymer sample in all particle size distribution was added and a stopwatch was operated at the same time. Then, the time taken until the vortex disappeared and a surface of liquid became completely horizontal was measured in seconds, and this was taken as the vortex time.

(6) Diaper absorbent core rewet (Rewet, g)

Absorbent bodies were manufactured using the super absorbent polymers of Examples and Comparative Examples using the following method, and rewet performance under pressure was evaluated. The preparation method of the absorbent body is as follows.

After uniformly applying 0.3 g of an adhesive (product name: FLC7228AZP, manufacturer: HB Fuller) (adhesive layer) on a first nonwoven fabric (product name: Softhann®, manufacturer: Sambo) of 350 mm * 100 mm in size with a hot melt sprayer, 4.5 g of the super absorbent polymer prepared in Preparation Example was uniformly applied thereto at a feed rate of 2 g/s to prepare a first application layer of the super absorbent polymer. 0.3 g of the adhesive was applied thereon in the same manner to prepare a first application layer of the adhesive. Thereafter, the process of preparing the first application layer of the super absorbent polymer and the first application layer of the adhesive was repeated two more time to produce a second application layer of the super absorbent polymer, a second application layer of the adhesive, a third application layer of the super absorbent polymer, and an adhesive layer. Then, an absorbent body was manufactured by attaching a second nonwoven fabric (product name: Softhann®, manufacturer: Sambo). The amount of adhesive used in each adhesive layer was the same as 0.3 g, and the amount of super absorbent polymer used in each super absorbent polymer layer was the same as 4.5 g.

The rewet performance of the absorbent body prepared above was evaluated by the following method.

85 mL of a 0.9 wt% sodium chloride aqueous solution (physiological saline) was injected into the center of each absorbent body (350 mm * 100 mm * 3 mm in size). 15 minutes later, a weight was placed on the absorbent body and an additional 85 mL of physiological saline was injected at the same location while applying a pressure of 0.42 psi. 15 minutes later, the weight on the absorbent body was temporarily removed and a new paper (filter paper of 300 gsm (S-300, Hankuk Paper), approximately 1.5 g($W_5$(g))/sheet) was placed on the absorbent body. Then, the weight was placed back on the paper to position the paper between the absorbent body and the weight. 2 minutes later, the amount of physiological saline emerged from the absorbent body to the paper was measured, and the rewet (g) was calculated according to the following Equation 3.

[Equation 3]

$$\text{Rewet (g)} = W_6(g) - W_5(g)$$

In Equation 3,
$W_5$ (g) is an initial weight of the paper, and $W_6$ (g) is a weight of the paper that absorbed the liquid emerged from the absorbent body for 2 minutes under a load (0.42 psi) after injecting physiological saline into the absorbent body under no pressure and under pressure.

[Table 1]

| | Bulk density (g/ml) | 16hr E/C (%) | CRC (g/g) | 0.7AUP (g/g) | Vortex (s) | Diaper rewet (g) |
|---|---|---|---|---|---|---|
| Example 1 | 0.58 | 7.0 | 33.4 | 19.8 | 28 | 0.83 |
| Example 2 | 0.6 | 7.7 | 38.7 | 12.5 | 24 | 0.70 |
| Example 3 | 0.6 | 9.1 | 32.5 | 18.2 | 26 | 0.89 |
| Example 4 | 0.61 | 9.3 | 32.7 | 16.0 | 23 | 0.94 |
| Comparati ve Example 1 | 0.49 | 7.8 | 32.0 | 17.0 | 23 | 1.52 |
| Comparati ve Example 2 | 0.52 | 8.0 | 34.0 | 16.2 | 22 | 1.72 |
| Comparati ve | 0.58 | 7.2 | 32.1 | 20.2 | 37 | 2.12 |
| Example 3 | | | | | | |
| Comparati ve Example 4 | 0.62 | 7.7 | 31.7 | 24.0 | 52 | 2.58 |

As shown in Table 1, in the case of the super absorbent polymer of one embodiment satisfying the absorption rate, bulk density, and 16hr EC, the rewet was excellent as 1.0 g or less, confirming that the super absorbent polymers of Examples had excellent rewet properties while exhibiting an equal or better absorption capacity and absorption rate compared to the super absorbent polymers of Comparative Examples.

## Claims

**1.** A super absorbent polymer comprising:

a base resin comprising a cross-linked polymer of an acrylic acid-based monomer having at least partially neutralized acidic groups and an internal cross-linking agent; and
a surface cross-linked layer formed by further cross-linking the cross-linked polymer using a surface cross-linking agent on the base resin,
wherein the super absorbent polymer satisfies the following conditions 1) to 4):

1) a centrifugal retention capacity (CRC) measured according to EDANA NWSP 241.0.R2 (15) is 32 g/g to 40 g/g,
2) an extractable content measured after swelling for 16 hours according to EDANA NWSP 270.0.R2 (15) is 10 wt% or less,
3) a vortex time by the vortex method is 30 seconds or less, and
4) a bulk density measured according to EDANA NWSP 251.0.R2 (15) is 0.55 g/ml or more and 0.65 g/ml or less.

**2.** The super absorbent polymer of Claim 1,
wherein the internal cross-linking agent is an epoxy compound or a polyethylene glycol-based polymer.

**3.** The super absorbent polymer of Claim 1,
comprising 10 wt% or less of particles having a particle diameter of 710 μm or more and 850 μm or less based on the super absorbent polymer.

4. The super absorbent polymer of Claim 1,
comprising 10 wt% or less of particles having a particle diameter of 150 μm or less based on the super absorbent polymer.

5. The super absorbent polymer of Claim 1,
wherein a rewet value of a pulpless absorbent body comprising the super absorbent polymer is 1 g or less.

6. A preparation method of a super absorbent polymer comprising:

a step of forming a hydrogel polymer by polymerizing a monomer composition containing an acrylic acid-based monomer having at least partially neutralized acidic groups, an internal cross-linking agent, an initiator, and hydrophobic particles (step 1);
a step of forming a base resin powder by drying the hydrogel polymer (step 2);
a first pulverization step of pulverizing the base resin powder to include 50 wt% or more of particles with a particle diameter of 710 μm or more (step 3);
a second pulverization step of pulverizing the base resin powder obtained after the first pulverization step to include 10 wt% or less of particles with a particle diameter of 710 μm or more and 10 wt% or less of particles with a particle diameter of 150 μm or less (step 4); and
a step of cross-linking the surface of the base resin in the presence of a surface cross-linking agent (step 5).

7. The preparation method of a super absorbent polymer of Claim 6,
wherein the internal cross-linking agent is an epoxy compound or a polyethylene glycol-based polymer.

8. The preparation method of a super absorbent polymer of Claim 6,
wherein the internal cross-linking agent is contained in an amount of 0.01 parts by weight or more based on 100 parts by weight of the acrylic acid-based monomer.

9. The preparation method of a super absorbent polymer of Claim 6, wherein

the initiator comprises a thermal polymerization initiator, and
the thermal polymerization initiator is contained in an amount of 0.001 to 0.2 parts by weight based on 100 parts by weight of the acrylic acid-based monomer.

10. The preparation method of a super absorbent polymer of Claim 6,
wherein a bulk density of the base resin powder obtained after the first pulverization step is 0.50 g/ml or less.

11. The preparation method of a super absorbent polymer of Claim 6,
wherein the super absorbent polymer satisfies the following conditions 1) to 4):

1) a centrifugal retention capacity (CRC) measured according to EDANA NWSP 241.0.R2 (15) is 32 g/g to 40 g/g,
2) an extractable content measured after swelling for 16 hours according to EDANA NWSP 270.0.R2 (15) is 10 wt% or less,
3) a vortex time by the vortex method is 30 seconds or less, and
4) a bulk density measured according to EDANA NWSP 251.0.R2 (15) is 0.55 g/ml or more and 0.65 g/ml or less.

12. A hygiene article comprising the super absorbent polymer of Claim 1.

**INTERNATIONAL SEARCH REPORT**

International application No.
**PCT/KR2023/018487**

**A. CLASSIFICATION OF SUBJECT MATTER**

**C08J 3/24**(2006.01)i; **C08J 3/12**(2006.01)i; **C08J 3/075**(2006.01)i; **C08F 220/04**(2006.01)i; **A61L 15/60**(2006.01)i

According to International Patent Classification (IPC) or to both national classification and IPC

**B. FIELDS SEARCHED**

Minimum documentation searched (classification system followed by classification symbols)

C08J 3/24(2006.01); A61L 15/22(2006.01); B29B 9/12(2006.01); C08F 2/00(2006.01); C08F 20/04(2006.01); C08F 20/06(2006.01); C08K 5/11(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 입경(particle diameter), 분쇄(pulverization), 고흡수성 수지(super absorbent polymer), 위생용품(sanitary product)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2020-0056050 A (LG CHEM, LTD.) 22 May 2020 (2020-05-22)<br>See claim 1; and paragraphs [0004]-[0111]. | 1-2,5,12<br>3-4,6-11 |
| Y | US 2012-0220745 A1 (MACHIDA, S. et al.) 30 August 2012 (2012-08-30)<br>See claim 1; and paragraph [0153]. | 3-4,6-11 |
| A | KR 10-2021-0093786 A (LG CHEM, LTD.) 28 July 2021 (2021-07-28)<br>See entire document. | 1-12 |
| A | KR 10-2021-0038378 A (LG CHEM, LTD.) 07 April 2021 (2021-04-07)<br>See entire document. | 1-12 |
| A | KR 10-2015-0059626 A (LG CHEM, LTD.) 01 June 2015 (2015-06-01)<br>See entire document. | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 February 2024** | **28 February 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2023/018487**

| Patent document cited in search report | Publication date (day/month/year) | Patent family member(s) | Publication date (day/month/year) |
|---|---|---|---|
| KR 10-2020-0056050 A | 22 May 2020 | CN 111511810 A | 07 August 2020 |
| | | CN 111511810 B | 12 May 2023 |
| | | EP 3708607 A1 | 16 September 2020 |
| | | EP 3708607 B1 | 07 June 2023 |
| | | JP 2021-509422 A | 25 March 2021 |
| | | US 11648531 B2 | 16 May 2023 |
| | | US 2020-0406228 A1 | 31 December 2020 |
| | | WO 2020-101150 A1 | 22 May 2020 |
| US 2012-0220745 A1 | 30 August 2012 | CN 102498134 A | 13 June 2012 |
| | | CN 102498134 B | 30 October 2013 |
| | | CN 102498135 A | 13 June 2012 |
| | | CN 102498135 B | 19 February 2014 |
| | | EP 2479195 A1 | 25 July 2012 |
| | | EP 2479195 B1 | 27 October 2021 |
| | | EP 2479196 A1 | 25 July 2012 |
| | | EP 2479196 B1 | 27 October 2021 |
| | | JP 5718816 B2 | 13 May 2015 |
| | | JP 5718817 B2 | 13 May 2015 |
| | | US 2012-0220733 A1 | 30 August 2012 |
| | | US 8513378 B2 | 20 August 2013 |
| | | US 9102804 B2 | 11 August 2015 |
| | | WO 2011-034146 A1 | 24 March 2011 |
| | | WO 2011-034147 A1 | 24 March 2011 |
| KR 10-2021-0093786 A | 28 July 2021 | BR 112021023193 A2 | 15 February 2022 |
| | | BR 112021023200 A2 | 26 July 2022 |
| | | CN 113454160 A | 28 September 2021 |
| | | CN 113454160 B | 02 May 2023 |
| | | CN 113748156 A | 03 December 2021 |
| | | CN 113748156 B | 07 November 2023 |
| | | CN 113767150 A | 07 December 2021 |
| | | CN 113767150 B | 24 November 2023 |
| | | CN 113785006 A | 10 December 2021 |
| | | EP 3907253 A1 | 10 November 2021 |
| | | EP 3943538 A1 | 26 January 2022 |
| | | EP 3943538 B1 | 23 August 2023 |
| | | EP 3943539 A1 | 26 January 2022 |
| | | EP 3943541 A1 | 26 January 2022 |
| | | JP 2022-521177 A | 06 April 2022 |
| | | JP 2022-531945 A | 12 July 2022 |
| | | JP 2022-533035 A | 21 July 2022 |
| | | JP 2022-533561 A | 25 July 2022 |
| | | JP 7297371 B2 | 26 June 2023 |
| | | JP 7321627 B2 | 07 August 2023 |
| | | JP 7321628 B2 | 07 August 2023 |
| | | JP 7337408 B2 | 04 September 2023 |
| | | KR 10-2021-0080186 A | 30 June 2021 |
| | | KR 10-2021-0093741 A | 28 July 2021 |
| | | KR 10-2021-0093742 A | 28 July 2021 |
| | | US 2022-0193634 A1 | 23 June 2022 |
| | | US 2022-0212166 A1 | 07 July 2022 |

Form PCT/ISA/210 (patent family annex) (July 2022)

**INTERNATIONAL SEARCH REPORT**
**Information on patent family members**

International application No.
**PCT/KR2023/018487**

| Patent document cited in search report | Publication date (day/month/year) | Patent family member(s) | Publication date (day/month/year) |
|---|---|---|---|
| | | US 2022-0242990 A1 | 04 August 2022 |
| | | US 2023-0241582 A1 | 03 August 2023 |
| | | WO 2021-125559 A1 | 24 June 2021 |
| | | WO 2021-125871 A1 | 24 June 2021 |
| | | WO 2021-125872 A1 | 24 June 2021 |
| | | WO 2021-150095 A1 | 29 July 2021 |
| KR 10-2021-0038378 A | 07 April 2021 | BR 112022002567 A2 | 03 May 2022 |
| | | CN 114144441 A | 04 March 2022 |
| | | EP 3985048 A1 | 20 April 2022 |
| | | JP 2022-542552 A | 05 October 2022 |
| | | JP 7330593 B2 | 22 August 2023 |
| | | US 2022-0274092 A1 | 01 September 2022 |
| | | WO 2021-066503 A1 | 08 April 2021 |
| KR 10-2015-0059626 A | 01 June 2015 | KR 10-1699504 B1 | 24 January 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220153918
- KR 1020230159227
- KR 20200128969


### Non-patent literature cited in the description


- **REINHOLD SCHWALM.** UV Coatings: Basics, Recent Developments and New Application. Elsevier, 2007, 115
- **ODIAN.** Principle of Polymerization. Wiley, 1981, 203